# EUROPÄISCHE PATENTSCHRIFT

(11) **EP 4 003 449 B1**
(45) Veröffentlichungstag und Bekanntmachung des Hinweises auf die Patenterteilung: **07.05.2025**
(21) Anmeldenummer: 20746621.0
(22) Anmeldetag: 24.07.2020
(51) Int. Cl.: A61M 1/16

(54) **DIALYSEGERÄT MIT EINER VORRICHTUNG ZUR IDENTIFIKATION EINES DIALYSATORS UND VERFAHREN ZUR IDENTIFIKATION EINES DIALYSATORS**
DIALYSIS MACHINE WITH A DEVICE FOR IDENTIFYING A DIALYZER AND METHOD FOR THE IDENTIFICATION OF A DIALYZER
MACHINE DE DIALYSE AVEC UN DISPOSITIF POUR IDENTIFIER UN DIALYSATEUR ET PROCÉDÉ D'IDENTIFICATION D'UN DIALYSATEUR

(30) Priorität: 25.07.2019 DE 102019120171
(43) Veröffentlichungstag der Anmeldung: 01.06.2022
(73) Patentinhaber: Fresenius Medical Care Deutschland GmbH, 61352 Bad Homburg (DE)
(72) Erfinder: FRENZEL, Alexander, 96166 Kirchlauter (DE)
(74) Vertreter: Herrmann, Uwe
(86) Internationale Anmeldenummer: PCT/EP2020/071032
(87) Internationale Veröffentlichungsnummer: WO 2021/014012

(56) Entgegenhaltungen:
- EP-B1- 2 919 829

## Beschreibung

Die vorliegende Erfindung betrifft ein Dialysegerät mit einem Dialysator, wobei das Dialysegerät eine Vorrichtung zur Identifikation des Dialysators aufweist, wobei die Vorrichtung Mittel zur Befüllung des Dialysators mit Flüssigkeit sowie Mittel zur Bestimmung der Füllmenge von Flüssigkeit aufweist, die in den Dialysator eingefüllt wird, und wobei die Vorrichtung des Weiteren Mittel zur Bestimmung des Drucks aufweist, der für den Druck in dem mit der Flüssigkeit befüllten Bereich des Dialysator repräsentativ ist.

Aus dem Stand der Technik bekannte Dialysegeräte weisen einen Dialysator auf, der eine von Dialysat durchströmte Dialysatseite und eine von Blut durchströmte Blutseite umfasst, die von einer oder mehreren semipermeablen Membranen voneinander getrennt sind. Während der Behandlung gelangen Schadstoffe aus dem Blut über diese Membran hinweg in das Dialysat, so dass das Blut dementsprechend eine Reinigung erfährt.

Die EP 2 919 829 B1 offenbart ein solches Dialysegerät.

Für die Behandlung eines Patienten wird ärztlich ein bestimmter Dialysator bzw. Dialysatortyp verordnet, der dann im Setup des Dialysegerätes eingestellt wird. Vor der Behandlung rüstet eine Hilfsschwester das Dialysgerät auf, setzt den Dialysator ein, schließt in an den Dialysatkreislauf an und eine andere Schwester bzw. der Arzt führt die Dialysebehandlung durch. Kommt es zu einer fehlerhaften Einstellung des genannten Setup oder wird trotz des an sich richtigen Setup ein falscher Dialysator eingesetzt, führt dies dazu, dass unter Umständen der Patient einer Gefährdung ausgesetzt ist, weil er nicht mit dem für ihn vorgesehenen Dialysatortyp behandelt wird. Abgesehen davon kann es zu einer Disruption des Dialysators kommen, was mit einer Kontamination des Dialysegerätes und ggf. mit einem Blutverlust des Patienten einhergehen kann.

Der vorliegenden Erfindung liegt daher die Aufgabe zugrunde, ein Dialysegerät und ein Verfahren bereitzustellen, mit dem sichergestellt werden kann, dass der korrekte Dialysator eingesetzt und für die Behandlung des Patienten verwendet wird.

Diese Aufgabe wird durch ein Dialysegerät mit den Merkmalen der Ansprüche 1 und 7 sowie durch ein Verfahren mit den Merkmalen der Ansprüche 10 und 13 gelöst.

Gemäß Anspruch 1 ist vorgesehen, dass eine Auswerteeinrichtung vorgesehen ist, die ausgebildet ist, die gemessene Füllmenge mit dem gemessenen Druck in Relation zu setzen und mit einer vorgegebenen Kennlinie oder mit einem vorgegebenen Kennwert zu vergleichen, wobei die Auswerteeinrichtung des Weiteren ausgebildet ist, ein Signal auszugeben, das davon abhängt, ob die festgestellte Relation (Füllmenge/Druck oder Druck/Füllmenge) mit der vorgegebenen Kennlinie bzw. mit dem vorgegebenen Kennwert übereinstimmt.

In diesem Fall wird die Größe bzw. der Typ des Dialysators beim Befüllen des Dialysators durch die eingesetzte Menge an Dialysat (oder einer sonstigen Flüssigkeit) und eine Druckmessung bestimmt.

Das Füllvolumen und der benötigte Druck führen entsprechend der Kennlinie für die Porengröße zu einer eindeutigen Identifikation des Dialysators. Anhand der Messungen ist eine Identifikation des Dialysators mit einer Toleranz von ± 2ml möglich.

Vorzugsweise wird ein Verhältnis aus Füllmenge/Druck gebildet und dieses Verhältnis mit einer Kennlinie oder mit einem Kennwert verglichen, der für den gewünschten Dialysatortyp charakteristisch ist. Die gemessene Füllmenge/Druck - Relation wird z.B. mit einem Datenbankeintrag zu den Eigenschaften des im Setup eingestellten Dialysators verglichen und somit plausibilisiert. Stimmt der Datenbankeintrag nicht mit der gemessenen Relation überein, kann eine Meldung oder sonstiges Signal erscheinen und der Nutzer zur Überprüfung der Einstellungen bzw. des eingesetzten Dialysators aufgefordert werden.

Wir kein Wert gemessen, kann die Meldung erscheinen, dass der Dialysator nicht befüllt werden konnte.

Die vorliegende Erfindung ist selbstverständlich ebenso realisierbar, wenn das Verhältnis von Druck/Füllvolumen gemessen und dieses Verhältnis mit einer Kennlinie oder mit einem Kennwert verglichen wird, der für den gewünschten Dialysatortyp charakteristisch ist.

Der im Rahmen dieser Erfindung verwendete Begriff "Druck" ist allgemein zu verstehen und umfasst nicht nur einen Druckwert als solchen, sondern alle daraus ableitbaren Größen, wie z.B. Druckverhältnis, eine Druckdifferenz, ein Mittelwert aus mehreren Druckwerten oder einen Druckverlauf über die Zeit etc.

Die Mittel zur Druckbestimmung können derart angeordnet sein, dass der Druck stromaufwärts und/oder stromabwärts und/oder in dem Dialysator gemessen wird.

Vorzugsweise ist der Dialysator mit dem Dialysatkreislauf des Dialysegerätes verbunden, so dass aus diesem Flüssigkeit in die Dialysatseite des Dialysators eingefüllt wird.

Vorzugsweise handelt es sich bei der Flüssigkeit, mit der der Dialysator gefüllt wird, um Dialysat, und besonders bevorzugt um das Dialysat, mit dem dann auch die Behandlung durchgeführt wird.

Vorzugsweise erfolgt jedoch die erfindungsgemäße Prüfung, ob der korrekte Dialysator eingesetzt wurde, vor Beginn der Behandlung des Patienten.

Die Mittel zur Druckbestimmung und die Auswerteeinrichtung können so ausgebildet sein, dass die Druckmessung bereits während und/oder nach der Befüllung des Dialysators erfolgt. Erfolgt die Druck- und Füllvolumenmessung während der Befüllung, kann ein Verhältnis aus diesen beiden Größen gebildet und über die Zeit ermittelt und mit einer entsprechenden Kennlinie verglichen werden.

Denkbar ist es aber auch, den Dialysator zu befüllen, vorzugsweise dessen Dialysatseite, und bei vollständiger Befüllung das Füllvolumen und den dann herrschenden Druck zu messen. Aus dem Verhältnis dieser beiden Größen und einem Vergleich mit einem Kennwert kann ebenfalls geprüft werden, ob der korrekte Dialysator verwendet wurde.

Die Druckmessung kann beispielsweise mittels Drucksensoren des Dialysegerätes erfolgen, die vorzugweise ohnehin am Dialysegerät vorgesehen sind.

Wie ausgeführt, handelt es sich bei der Flüssigkeit vorzugsweise um Dialysat.

Bei den Mitteln zur Befüllung des Dialysators mit Flüssigkeit kann es sich um die Dialysatpumpe des Dialysegerätes handeln. Dies kann eine rotatorisch arbeitende Pumpe, wie z.B. eine peristaltische Pumpe sein, so dass die dem Dialysator zugeführte Menge an Dialysat über die Pumpenrotation bestimmt werden kann. Ein eigens für die Ermittlung der Füllmenge vorgesehener Sensor ist in diesem Fall nicht erforderlich.

Wie ausgeführt, ist vorzugsweise vorgesehen, dass die Mittel zur Befüllung des Dialysators mit der Dialysatseite des Dialysators in Fluidverbindung stehen.

In einer weiteren Ausführung betrifft die vorliegende Erfindung ein Dialysegerät mit einem Dialysator, wobei das Dialysegerät eine Vorrichtung zur Identifikation des Dialysators aufweist.

Dabei ist vorgesehen, dass der Dialysator über ein elektrisch leitendes Element verfügt und dass die Vorrichtung ausgebildet ist, den elektrischen Widerstand des leitenden Elementes zu messen und dass eine Auswerteeinrichtung vorgesehen ist, die ausgebildet ist, den gemessenen Widerstand mit einem vorgegebenen Widerstand zu vergleichen, wobei die Auswerteeinrichtung des Weiteren ausgebildet ist, ein Signal auszugeben, das davon abhängt, ob der gemessene Widerstand mit dem vorgegebenen Widerstand übereinstimmt.

Diese Ausführungsform kann zusätzlich zu der eingangs beschriebenen ersten Ausführungsform vorhanden sein.

Denkbar ist es beispielsweise, die Identifikation des Dialysators mittels der Messung des Widerstands durchzuführen und als Fall-Back Lösung die Messung von Druck und Füllvolumen vorzunehmen, wie das eingangs beschrieben wurde. Auch eine umgekehrte Vorgehensweise ist denkbar und von der Erfindung umfasst.

Das elektrisch leitende Element kann in einen Aufkleber integriert sein, der vorzugsweise an einer Kupplung des Dialysators angeordnet ist.

Denkbar ist es, z.B. an der Dialysatorkupplung nach dem Rasten, d.h. nach deren Verbindung mit dem Gegenstück des Dialyatkreislaufs, einen Aufkleber mit einem integrierten Metallstreifen aufzubringen, der über einen festgelegten Messwiderstand verfügt. Grundsätzlich kann dieser Aufkleber auch an anderer Stelle als an der Dialysatorkupplung befestigt werden. Der Aufkleber kann gerade, gekrümmt, ringförmig etc. ausgebildet sein.

Bevorzugt ist es, wenn die Vorrichtung zwei Litzen am Dialysatzulaufschlauch oder - ablaufschlauch aufweist, die mit dem leitenden Element in leitendem Kontakt stehen, um dessen Widerstand zu messen. Vorzugweise ist der Abstand zwischen den beiden Messlitzen bzw. -stiften immer gleich, daher kann die Messung unabhängig von der Position des Zulaufs an der Kupplung stattfinden.

Das Dialysegerät weist vorzugsweise einen Dialysatkreislauf auf, an den der Dialysator vorzugsweise zu dessen Identifikation angeschlossen ist und in Fluidverbindung steht. Dies gilt für beide der erfindungsgemäßen Ausführungsformen.

Der gemessene elektrische Widerstand wird mit einem Kennwert des korrekten Dialysators z.B. aus einer Datenbank verglichen und somit plausibilisiert. Wird ein abweichender Wert gemessen, kann eine Meldung oder ein sonstiges Signal den Nutzer auffordern, die Einstellungen bzw. den Dialysatortyp zu überprüfen. Wird kein Wert gemessen, wie z.B. bei einem Fremddialysator oder bei einem Altbestand, kann eine Meldung erscheinen, dass die Messung nicht durchgeführt werden kann.

Die vorliegende Erfindung betrifft des Weiteren ein Verfahren zur Identifikation eines Dialysators, wobei der Dialysator mit Flüssigkeit befüllt wird, die Füllmenge an Flüssigkeit in dem Dialysator sowie der Druck bestimmt werden, der für den Druck in befüllten Bereich des Dialysator repräsentativ ist, und die gemessene Füllmenge mit dem gemessenen Druck in Relation gesetzt werden und mit einer vorgegebenen Kennlinie oder mit einem vorgegebenen Kennwert verglichen werden, wobei ein Signal ausgegeben wird, das davon abhängt, ob die festgestellte Relation mit der vorgegebenen Kennlinie bzw. mit dem vorgegebenen Kennwert übereinstimmt.

Wie ausgeführt, kann der Druck stromaufwärts und/oder stromabwärts und/oder in dem Dialysator gemessen werden.

Die Druckmessung kann während und/oder nach der Befüllung des Dialysators erfolgen.

Vorzugsweise wird zum Zwecke der Identifikation des Dialysators dessen Dialysatseite mit Flüssigkeit, vorzugsweise mit Dialysat befüllt.

Das Verfahren zur Identifikation eines Dialysators kann zusätzliche Merkmale aufweisen, wobei der Dialysator über ein leitendes Element verfügt und der elektrische Widerstand dieses leitenden Elementes gemessen und mit einem vorgegebenen Widerstand verglichen wird, wobei ein Signal ausgegeben wird, das davon abhängt, ob der gemessene Widerstand mit dem vorgegebenen Widerstand übereinstimmt.

Dabei ist es denkbar, dass der Dialysator mit einem Aufkleber versehen wird, der das leitende Element aufweist oder aus diesem besteht.

Die Messung des Widerstands kann mit Hilfe von zwei Litzen, Stiften oder dergleichen am Dialysatzulaufschlauch oder -ablaufschlauch vorgenommen werden, die mit dem leitenden Element in leitendem Kontakt stehen, um dessen Widerstand zu messen.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

Weitere Einzelheiten und Vorteile der Erfindung werden anhand eines im Folgenden beschriebenen Ausführungsbeispiels näher erläutert.

In einer ersten Ausführungsform der Erfindung wird der Dialysator vor der Behandlung des Patienten an den Dialysatkreislauf des Dialysegerätes angeschlossen und die Dialysatpumpe wird in Betrieb gesetzt. Zeitgleich wird der Druck an der Einlassseite und/oder an der Auslassseite des Dialysators gemessen und ein Verhältnis aus dem bereits in den Dialysator eingelaufenen Volumen an Dialysat und z.B. dem einlassseitigen Druck gebildet. Dieses Verhältnis kann laufend oder zu bestimmten Zeitabständen gebildet werden.

Es wird verglichen mit einer Kennlinie oder Kennwerten, die ein solches Verhältnis für den gewünschten Dialysator darstellt bzw. sind.

Stellt eine Auswerteeinrichtung fest, dass das gemessene Verhältnis dieser Kennlinie bzw. dem Kennwert entspricht, erfolgt keine Signalisierung an den Nutzer oder die Mitteilung, dass der eingesetzte Dialysatortyp in Ordnung ist. Stellt die Auswerteeinrichtung jedoch fest, dass dies nicht der Fall ist, wird ein Signal ausgegeben, aus dem der Nutzer entnehmen kann, dass nicht der richtige Dialysatortyp verwendet wurde. Denkbar ist in diesem Fall auch, dass das Dialysegerät die Behandlung verhindert, in dem z.B. die Pumpen gestoppt und Ventile geschlossen werden.

Die oben beschriebene Prüfung der Identität des Dialysators findet vorzugsweise vor der Dialysebehandlung des Patienten statt. Vorzugsweise ist die Blutpumpe noch nicht in Betrieb, so dass bei der erfindungsgemäßen Prüfung keinerlei Beeinflussung bzw. Änderung der Blutzusammensetzung des Patienten stattfindet.

In einer zweiten Ausführungsform weist der Dialysator zusätzlich eine elektrisch leitende Fläche, Leiterbahn etc. auf, die in dem Dialysator integriert sein kann oder z.B. durch einen Aufkleber auf diesen aufgebracht werden kann.

Eine Messeinrichtung misst den elektrischen Widerstand der elektrisch leitenden Fläche, Leiterbahn etc. und vergleicht diesen mit einem Kennwert, der für den gewünschten Dialysator charakteristisch ist.

Stellt eine Auswerteeinrichtung fest, dass der gemessene Wert diesem Kennwert entspricht, erfolgt keine Signalisierung an den Nutzer oder die Mitteilung, dass der eingesetzte Dialysatortyp in Ordnung ist. Stellt die Auswerteeinrichtung jedoch fest, dass dies nicht der Fall ist, wird ein Signal ausgegeben, aus dem der Nutzer entnehmen kann, dass nicht der richtige Dialysatortyp verwendet wurde. Denkbar ist in diesem Fall auch, dass das Dialysegerät die Behandlung verhindert, in dem z.B. die Pumpen gestoppt und Ventile geschlossen werden.

Die vorliegende Erfindung kann nur mit der ersten Ausführungsform oder mit der ersten und der zweiten Ausführungsformen umgesetzt werden. Im letzten Fall kann die Füllvolumen/Druck-Messung als Fall-Back verwendet werden, wenn die Messung des elektrischen Widerstands nicht funktioniert, etwa weil es sich um einen Fremd-Dialysator, einen defekten Aufkleber, einen Dialysator aus einem Restbestand etc. handelt. Es kann aber auch vorgesehen sein, dass mit Hilfe der Messung des elektrischen Widerstandes ein Dialysatortyp identifizierbar wird und ihm mit Hilfe der Füllvolumen/Druckmessung eine zugehörige Dialysatorgröße zugeordnet wird. Mit Hilfe einer solchen kombinierten Methode wird eine besonders große Vielzahl von unterschiedlichen Dialysatoren identifizierbar.

Durch die vorliegende Erfindung können je nach Ausführungsform die folgenden Vorteile erzielt werden:
- Verbesserung der Patientensicherheit durch Verringerung des Risikos einer fehlerhaften Behandlung durch den Benutzer.
- Risiko einer Disruption und damit verbundener Geräte-Kontamination wird verringert, es kommt zu keinem Blutverlust des Patienten.
- Umsetzung auf verschiedenen Dialysemaschinen und Produktfamilien möglich.
- Kreuz-Kompatibilität wird sichergestellt, Altbestände von Dialysatoren können weiterverwendet werden.
- Servicequalität steigt durch neue Schutzmaßnahme, der Benutzer fühlt sich sicherer.
- Bei der Ausführungsform mit Füllvolumen- und Druckmessung: Auch Identifikation und Plausibilisierung von Fremd -Dialysatoren möglich.
- Bei der Ausführungsform mit Füllvolumen- und Druckmessung: Günstige Umsetzung in der Entwicklung, da auf bestehende Funktionen und Methoden zurückgegriffen werden kann.
- Bei Ausführungsform mit Widerstandsmessung: Erhöhte Qualität und Alleinstellungsmerkmal.
- Bei Ausführungsform mit Widerstandsmessung: Bestehender Produktionsablauf der Dialysatoren bleibt unverändert und wird nur erweitert, indem der Aufkleber, z.B. in Form eines Rings, der sich um die Dialysatorkupplung oder ein anderes Teil des Dialysators erstreckt, aufgeklebt wird.

## Patentansprüche

1. Dialysegerät mit einem Dialysatkreislauf und mit einem Dialysator, wobei das Dialysegerät eine Vorrichtung zur Identifikation des Dialysators aufweist, wobei die Vorrichtung Mittel zur Befüllung des Dialysators mit Flüssigkeit sowie Mittel zur Bestimmung der Füllmenge von Flüssigkeit aufweist, die in den Dialysator eingefüllt wird, und wobei die Vorrichtung des Weiteren Mittel zur Bestimmung des Drucks aufweist, der für den Druck in befüllten Bereich des Dialysator repräsentativ ist, **dadurch gekennzeichnet, dass** das Dialysegerät eine Auswerteeinrichtung aufweist, die ausgebildet ist, die gemessene Füllmenge mit dem gemessenen Druck in Relation zu setzen und mit einer vorgegebenen Kennlinie oder mit einem vorgegebenen Kennwert zu vergleichen, wobei die Auswerteeinrichtung des Weiteren ausgebildet ist, ein Signal auszugeben, das davon abhängt, ob die festgestellte Relation mit der vorgegebenen Kennlinie bzw. mit dem vorgegebenen Kennwert übereinstimmt.

2. Dialysegerät nach Anspruch 1, **dadurch gekennzeichnet, dass** es sich bei dem gemessenen Druck um ein Druckverhältnis oder um einen Druckverlauf handelt.

3. Dialysegerät nach Anspruch 1 oder 2, **dadurch gekennzeichnet, dass** die Mittel zur Druckbestimmung derart angeordnet sind, dass der Druck bzw. der Druckverlauf stromaufwärts und/oder stromabwärts und/oder in dem Dialysator gemessen wird.

4. Dialysegerät nach einem der vorhergehenden Ansprüche, **dadurch gekennzeichnet, dass** die Mittel zur Druckbestimmung und die Auswerteeinrichtung so ausgebildet sind, dass die Druckmessung während und/oder nach der Befüllung des Dialysators erfolgt.

5. Dialysegerät nach einem der vorhergehenden Ansprüche, **dadurch gekennzeichnet, dass** es sich bei der Flüssigkeit um Dialysat handelt und/oder dass es sich bei den Mitteln zur Befüllung des Dialysators mit Flüssigkeit um die Dialysatpumpe des Dialysegerätes handelt.

6. Dialysegerät nach einem der vorhergehenden Ansprüche, **dadurch gekennzeichnet, dass** der Dialysator eine Dialysat- und eine von dieser durch eine Membran abgetrennte Blutseite aufweist und dass die Mittel zur Befüllung des Dialysators mit der Dialysatseite des Dialysators in Fluidverbindung stehen.

7. Dialysegerät nach einem der vorhergehenden Ansprüche, **dadurch gekennzeichnet, dass** der Dialysator an den Dialysatkreislauf angeschlossen ist und über ein leitendes Element verfügt und dass die Vorrichtung ausgebildet ist, den elektrischen Widerstand des leitenden Elementes zu messen und dass eine Auswerteeinrichtung vorgesehen ist, die ausgebildet ist, den gemessenen Widerstand mit einem vorgegebenen Widerstand zu vergleichen, wobei die Auswerteeinrichtung des Weiteren ausgebildet ist, ein Signal auszugeben, das davon abhängt, ob der gemessene Widerstand mit dem vorgegebenen Widerstand übereinstimmt.

8. Dialysegerät nach Anspruch 7, **dadurch gekennzeichnet, dass** das leitende Element in einen Aufkleber integriert ist, der vorzugsweise an einer Kupplung des Dialysators angeordnet ist.

9. Dialysegerät nach Anspruch 7 oder 8, **dadurch gekennzeichnet, dass** die Vorrichtung zwei Litzen am Dialysatzulaufschlauch oder -ablaufschlauch aufweist, die mit dem leitenden Element in leitendem Kontakt stehen, um dessen Widerstand zu messen.

10. Verfahren unter Verwendung eines Dialysegeräts nach einem der vorhergehenden Ansprüche zur Identifikation eines Dialysators, **dadurch gekennzeichnet, dass** der Dialysator mit Flüssigkeit befüllt wird, die Füllmenge an Flüssigkeit in dem Dialysator sowie der Druck bestimmt werden, der für den Druck in befüllten Bereich des Dialysator repräsentativ ist, und die gemessene Füllmenge mit dem gemessenen Druck in Relation gesetzt werden und mit einer vorgegebenen Kennlinie oder mit einem vorgegebenen Kennwert verglichen werden, wobei ein Signal ausgegeben wird, das davon abhängt, ob die festgestellte Relation mit der vorgegebenen Kennlinie bzw. mit dem vorgegebenen Kennwert übereinstimmt.

11. Verfahren nach Anspruch 10, **dadurch gekennzeichnet, dass** der Druck stromaufwärts und/oder stromabwärts und/oder in dem Dialysator gemessen wird und/oder dass die Druckmessung während und/oder nach der Befüllung des Dialysators erfolgt.

12. Verfahren nach einem der Ansprüche 10 oder 11 **dadurch gekennzeichnet, dass** zum Zwecke der Identifikation des Dialysators dessen Dialysatseite mit Flüssigkeit, vorzugsweise mit Dialysat befüllt wird.

13. Verfahren nach einem der Ansprüche 10 bis 12, **dadurch gekennzeichnet,**
**dass** der Dialysator über ein leitendes Element verfügt und der elektrische Widerstand dieses leitenden Elementes gemessen und mit einem vorgegebenen Widerstand verglichen wird, wobei ein Signal ausgegeben wird, das davon abhängt, ob der gemessene Widerstand mit dem vorgegebenen Widerstand übereinstimmt.

14. Verfahren nach Anspruch 13, **dadurch gekennzeichnet, dass** der Dialysator mit einem Aufkleber versehen wird, der das leitende Element aufweist oder aus diesem besteht.

15. Verfahren nach Anspruch 13 oder 14, **dadurch gekennzeichnet, dass** die Messung des Widerstands mit Hilfe von zwei Litzen am Dialysatzulaufschlauch oder -ablaufschlauch vorgenommen wird, die mit dem leitenden Element in leitendem Kontakt stehen, um dessen Widerstand zu messen.

## Claims

1. Dialysis machine having a dialyzate circuit and having a dialyzer, wherein the dialysis machine has an apparatus for identifying the dialyzer; wherein the apparatus has means for filling the dialyzer with fluid and has means for determining the filling amount of fluid that is filled into the dialyzer; and wherein the apparatus furthermore has means for determining the pressure that is characteristic for the pressure in the filled region of the dialyzer, **characterized in that** the dialysis machine has an evaluation device that is configured to put the measured filling amount into relationship with the measured value and to compare it with a predefined characteristic curve or with a predefined characteristic value, with the evaluation device furthermore being configured to output a signal that depends on whether the relationship determined coincides with the predefined characteristic curve or with the predefined characteristic value.

2. Dialysis machine in accordance with claim 1, **characterized in that** the measured pressure is a pressure ratio or a pressure development.

3. Dialysis machine in accordance with claim 1 or claim 2, **characterized in that** the means for the pressure determination are arranged such that the pressure or the pressure development is measured upstream and/or downstream of and/or in the dialyzer.

4. Dialysis machine in accordance with one of the preceding claims, **characterized in that** the means for the pressure determination and the evaluation device are configured such that the pressure measurement takes place during and/or after the filling of the dialyzer.

5. Dialysis machine in accordance with one of the preceding claims, **characterized in that** the fluid is dialyzate; and/or **in that** the means for filling the dialyzer with fluid is the dialyzate pump of the dialysis machine.

6. Dialysis machine in accordance with one of the preceding claims, **characterized in that** the dialyzer has a dialyzate side and a blood side separated therefrom by a membrane; and **in that** the means for filling the dialyzer are in fluid communication with the dialyzate side of the dialyzer.

7. Dialysis machine in accordance with one of the preceding claims, **characterized in that** the dialyzer is connected to the dialyzate circuit has an electrically conductive element; and **in that** that the apparatus is configured to measure the electrical resistance of the conductive element; and **in that** an evaluation device is provided that is configured to compare the measured resistance with a predefined resistance, with the evaluation device furthermore being configured to output a signal that depends on whether the measured resistance coincides with the predefined resistance.

8. Dialysis machine in accordance with claim 7, **characterized in that** the conductive element is integrated in an adhesive label that is preferably arranged at a coupling of the dialyzer.

9. Dialysis machine in accordance with claim 7 or claim 8, **characterized in that** the apparatus has two strands at the dialyzate inflow tube or dialyzate outflow tube that are in conductive contact with the conductive element to measure its resistance.

10. Method of identifying a dialyzer using a dialysis machine in accordance with one of the preceding claims, **characterized in that** the dialyzer is filled with fluid, the filling amount of fluid in the dialyzer and the pressure that is representative for the pressure in the filled region of the dialyzer are determined, and the measured filling amount is put into relationship with the measured pressure and is compared with a predefined characteristic curve or with a predefined characteristic value, with a signal being output that depends on whether the relationship determined coincides with the predefined characteristic curve or with the predefined characteristic value.

11. Method in accordance with claim 10, **characterized in that** the pressure is measured upstream and/or downstream and/or in the dialyzer; and/or **in that** the pressure measurement takes place during and/or after the filling of the dialyzer.

12. Method in accordance with one of the claims 10 or 11, **characterized in that** the dialyzate side of the dialyzer is filled with fluid, preferably with dialyzate, for the purpose of identifying the dialyzer.

13. Method in accordance with one of the claims 10 to 12, **characterized in that** the dialyzer has a conductive element and the electrical resistance of this conductive element is measured and is compared with a predefined resistance, with a signal being output that depends on whether the measured resistance coincides with the predefined resistance.

14. Method in accordance with claim 13, **characterized in that** the dialyzer is provided with an adhesive label that comprises or consists of the conductive element.

15. Method in accordance with claim 13 or claim 14, **characterized in that** the measurement of the resistance is carried out with the aid of two strands at the dialyzate inflow tube or dialyzate outflow tube that are in conductive contact with the conductive element to measure its resistance.

## Revendications

1. Appareil de dialyse avec un circuit de dialysat et avec un dialyseur, l'appareil de dialyse présentant un dispositif pour l'identification du dialyseur, le dispositif présentant des moyens pour remplir le dialyseur avec du liquide ainsi que des moyens pour déterminer la quantité de remplissage de liquide qui est introduite dans le dialyseur, et le dispositif présentant en outre des moyens pour déterminer la pression qui est représentative de la pression dans la zone remplie du dialyseur, **caractérisé en ce que** l'appareil de dialyse présente un dispositif d'évaluation qui est réalisé pour mettre en relation la quantité de remplissage mesurée avec la pression mesurée et pour la comparer à une courbe caractéristique prédéfinie ou à une valeur caractéristique prédéfinie, le dispositif d'évaluation étant en outre réalisé pour émettre un signal qui dépend de si la relation constatée correspond à la courbe caractéristique prédéfinie ou à la valeur caractéristique prédéfinie.

2. Appareil de dialyse selon la revendication 1, **caractérisé en ce que** la pression mesurée consiste en un rapport de pression ou une courbe de pression.

3. Appareil de dialyse selon la revendication 1 ou 2, **caractérisé en ce que** les moyens de détermination de la pression sont agencés de telle sorte que la pression ou la courbe de pression est mesurée en amont et/ou en aval et/ou dans l'appareil de dialyse.

4. Appareil de dialyse selon l'une quelconque des revendications précédentes, **caractérisé en ce que** les moyens de détermination de la pression et le dispositif d'évaluation sont réalisés de telle sorte que la mesure de la pression s'effectue pendant et/ou après le remplissage du dialyseur.

5. Appareil de dialyse selon l'une quelconque des revendications précédentes, **caractérisé en ce que** le liquide consiste en du dialysat et/ou **en ce que** les moyens de remplissage du dialyseur en liquide consistent en la pompe à dialysat de l'appareil de dialyse.

6. Appareil de dialyse selon l'une quelconque des revendications précédentes, **caractérisé en ce que** le dialyseur présente un côté dialysat et un côté sang séparé de celui-ci par une membrane et **en ce que** les moyens de remplissage du dialyseur sont en communication fluidique avec le côté dialysat du dialyseur.

7. Appareil de dialyse selon l'une quelconque des revendications précédentes, **caractérisé en ce que** le dialyseur est raccordé au circuit de dialysat et dispose d'un élément conducteur et **en ce que** le dispositif est réalisé pour mesurer la résistance électrique de l'élément conducteur et **en ce qu'**il est prévu un dispositif d'évaluation qui est réalisé pour comparer la résistance mesurée à une résistance prédéfinie, le dispositif d'évaluation étant en outre réalisé pour émettre un signal qui dépend de si la résistance mesurée correspond à la résistance prédéfinie.

8. Appareil de dialyse selon la revendication 7, **caractérisé en ce que** l'élément conducteur est intégré dans une étiquette adhésive, qui est de préférence agencée sur un accouplement du dialyseur.

9. Appareil de dialyse selon la revendication 7 ou 8, **caractérisé en ce que** le dispositif présente deux fils sur le tuyau d'entrée ou de sortie du dialysat qui sont en contact conducteur avec l'élément conducteur afin de mesurer sa résistance.

10. Procédé utilisant un appareil de dialyse selon l'une quelconque des revendications précédentes pour identifier un dialyseur, **caractérisé en ce que** le dialyseur est rempli de liquide, la quantité de remplissage de liquide dans le dialyseur et la pression qui est représentative de la pression dans la zone remplie du dialyseur sont déterminées, et la quantité de remplissage mesurée est mise en relation avec la pression mesurée et comparée à une courbe caractéristique prédéfinie ou à une valeur caractéristique prédéfinie, un signal étant émis, qui dépend de si la relation constatée correspond à la courbe caractéristique prédéfinie ou à la valeur caractéristique prédéfinie.

11. Procédé selon la revendication 10, **caractérisé en ce que** la pression est mesurée en amont et/ou en aval et/ou dans le dialyseur et/ou **en ce que** la mesure de la pression est effectuée pendant et/ou après le remplissage du dialyseur.

12. Procédé selon l'une quelconque des revendications 10 ou 11, **caractérisé en ce que**, dans le but d'identifier le dialyseur, son côté dialysat est rempli de liquide, de préférence de dialysat.

13. Procédé selon l'une quelconque des revendications 10 à 12, **caractérisé en ce que** le dialyseur dispose d'un élément conducteur et **en ce que** la résistance électrique de cet élément conducteur est mesurée et comparée à une résistance prédéfinie, un signal étant émis, qui dépend de si la résistance mesurée correspond à la résistance prédéfinie.

14. Procédé selon la revendication 13, **caractérisé en ce que** le dialyseur est muni d'une étiquette adhésive présentant l'élément conducteur ou constituée de celui-ci.

15. Procédé selon la revendication 13 ou 14, **caractérisé en ce que** la mesure de la résistance est effectuée à l'aide de deux fils sur le tube d'entrée ou de sortie du dialysat, qui sont en contact conducteur avec l'élément conducteur afin de mesurer sa résistance.
